# EUROPEAN PATENT APPLICATION

(11) **EP 0 829 259 A1**
(43) Date of publication of application: **18.03.1998**
(21) Application number: 97306637.6
(22) Date of filing: 29.08.1997
(51) Int. Cl.: A61K 7/15

(54) **Foam/gel with microbeads and/or fine particles**

(30) Priority: 04.09.1996 US 707547
(71) Applicant: WARNER-LAMBERT COMPANY, Morris Plains New Jersey 07950 (US)
(72) Inventor: Geria, Navin Manohar, Warren, New Jersey 07060 (US); Melillo, Joseph, Fuchu-shi, Tokyo 183 (JP)
(74) Representative: Powell, Timothy John

(57) **Abstract**

The present invention is directed to foams and gels for personal hygiene use which contain beads having an active ingredient, fine particles such as powders, or a combination of both beads and powders. The beads may be microencapsulated and contain any one or more of a variety of ingredients, while the powders may be of a suitable form to cause mild abrasion to remove dead skin cells.

## Description

This invention relates to foams and gels such as shaving creams for personal hygiene use and, in particular, to such foams and gels containing microbeads, fine particles or a combination of both.

Foams and gels for personal hygiene are well known. Among the many types of such foams and gels are shaving creams, pre-shave compositions, shampoos and liquid soaps. Such foams and gels are delivered to the user via a number of different, known methods, including aerosol sprays, so called "post-foaming" gels such as disclosed in US Patent No. 3,541,581, pump sprays and other non-aerosol sprays. Each of these various foams and gels contain a number of ingredients, both active and inactive, which provide the desired objective of the product. However, none of the known foams or gels contain beads which contain active ingredients or powders which provide abrasion to the skin.

The present invention is directed to foams and gels for personal hygiene use which contain beads having an active ingredient, fine particles such as powders, or a combination of both beads and powders. The beads may be microencapsulated and contain any one or more of a variety of ingredients, while the powders may be of a suitable form to cause mild abrasion to remove dead skin cells.

Accordingly, it is an advantage of the present invention to provide a foam, gel or post-foaming gel which contains beads having an active material, powder which provides abrasion to the skin or a combination of both.

There now follows a description of a preferred embodiment of the invention, by way of example.

Skin cells are formed and naturally replaced during a cycle which encompasses approximately 21 days. Consequently, a large number of dead skin cells are constantly building up on the surface of the skin. In addition, various other debris, such as that caused by air pollution, also accumulates on the surface of the skin. This accumulation of debris prevents the skin's freshest, newest layer from being exposed and consequently dulls the complexion. Numerous forms of shaving creams, gels and post-foaming gels, both aerosol and non-aerosol, for shaving are known in the art. For example, US Patent No. 3,541,581 issued to Monson and hereby incorporated by reference, describes post-foaming gels and a package for containing such gels. Virtually all of these products which are commercially available provide the benefits of moistening the beard and lubricating the skin before shaving. None of these known products, however, address the problems of dead skin cell build up or the accumulation of debris on the face.

The addition of microparticles in the form of beads, powders or a combination of both, to the shaving cream or gel, addresses the problem of an accumulation of debris, including dead skin cells, on the surface of the skin. The microparticles act to scrub off the accumulation of debris on the skin and act in a uniform and fast manner because heavy scrubbing is not required. The addition of microparticles to a post-foaming gel offers particular benefits in that upon application it provides gentle, deep cleaning to the skin. The microparticles help weaken and loosen the dead, dry skin cells and other debris and the washing portion of shaving washes the debris from the skin. Consequently, the skin is retextured and fresher and will lead to future shaves being closer and more comfortable because there will be fewer lumps and bumps caused by debris for the razor to catch on the nick.

The formula for the cream or gel may be any formula known in the art or contemplated by one in the art. For example, various formulas are commercialized by numerous companies, including the Vitamins and Fine Chemicals Group of Hoffmann-Laroche, Nutley, New Jersey, Aqualon Company of Wilmington, Delaware, R.T. Vanderbilt Company, Inc. of Norwalk, Connecticut, Eastman Chemical Company of Kingsport, Tennessee, and Florasynth Inc. of Teterboro, New Jersey. None of the currently available formulas for shaving creams or gels, however, include powder, microbeads or a combination of both.

The addition of powders to a shaving cream or gel formula provides the user with the benefit of a mechanism for enhancing the exfoliation of dead skin cells through abrasion. In addition, the powder also acts to remove any other debris, such as that caused by air pollution, which has accumulated on the skin. The removal of such debris and the consequent unplugging of skin pores can create a healthier skin and prevent such diseases as acne. Further, the removal of the debris enhances the individual's complexion in that it exposes the newest, freshest layer of skin.

The powders, microbeads or combination thereof may be added in virtually any proportion to any shaving cream or gel formula. Many different powder materials are suitable for inclusion in a shaving cream. Such materials include crushed nut shells (preferably walnut or almond shells), crushed apricot seeds, loofah, talc, magnesium stearate, chalk, kaolin, zinc oxide, titanium oxide, coated powders, beads, micro-silica beads (preferably those with 90/10 ratio), boron nitride, zinc stearate, magnesium oxide, cellulose powders, nylon powder or polyamid resin, silk powder protein, silk coated pigments, calcium carbonate, magnesium carbonate, mica, sericite or combinations thereof. In addition, organic plastic powders, such as polymethylmethacrylate beads, polyethylene beads and micropowders, polyvinylidene talc, polytetrafluoroethylene (teflon), polyamides, polyurethane, polystyrene or combinations thereof may be employed. While the powders may be readily obtained from many sources, Lipo Chemicals, Inc. of Paterson, New Jersey provides many powders in the above list in an acceptable form.

Other possible powder materials are synthetic clay, including magnesium lithium sodium silicate and silica, including silicon oxide spheres. A preferred embodiment of the silicon oxide spheres comprises spheres which are size 7 to 14 microns, with a surface area of 200-400 square meters/gram and a density of 36-72 gram/liter. The other preferred microbeads and powders may if possible be of a similar particle size. The listed powder materials may be used in any desired ratio, mixture or concentration. In addition, the powder materials may be used in any combination and any listed powder material may be used together with any other listed powder material.

In order to provide additional properties to the shaving cream or gel formula, microencapsulated particles may also be included in the formula. The particles may contain various active or inactive ingredients which provide the formula with certain properties, such as enhanced lubrication or fragrance. One such type of microparticle is alginate microspheres as provided by Lipo Technologies, Inc of Vandalia, Ohio. Such microparticles may also be in the form of microcapsules. The microcapsules basically comprise a core material of hydrophobic ingredients surrounded by a capsule wall which protects the core from the surroundings, while the microspheres comprises a hydrogel matrix which encapsulates the core material. In either version, the core material may comprise any active or inactive material, such as a lubricant, which is activated upon exposure to water during shaving. Additional materials, such as jojoba oil, mineral oil, sesame oil, silicone oil, aloe vera oil, tocopheryl acetate, fragrance, vitamin E, dimethicone, allantoin, lanolin, sodium PCA, color, vitamin A, vitamin C, glycolic acid, glycerin, petrolatum, propane, isobutane or combinations thereof may also be employed within the microparticles, with the ratio and mixture dependent upon the desired properties of the final product.

Another form of microparticle which may be included in the formula is pearlescent beads which contain active ingredients. Preferably, the beads are crushable such that during the application of the shaving cream or gel to the face the beads are crushed, thereby releasing their active material to the skin. One example of pearlescent beads is LipoPearl Ô as commercialized by Lipo Technologies, Inc. Various materials may be included within the beads, including jojoba oil, mineral oil, sesame oil, silicon oil, aloe vera oil, tocopheryl acetate, fragrance, vitamin E, dimethicone, allantoin, lanolin, sodium PCA, color, vitamin A, vitamin C, glycolic acid, glycerin, petrolatum, propane, isobutane or combinations thereof. Another example of beads which may be included in the formula are Florabeads® as commercialized by International Flora Technologies, Ltd. of Apache Junction, Arizona. The Florabeads® contain hydrogenated jojoba oil which acts as an exfoliant.

While there have been described what are presently believed to be the preferred embodiments of the invention, those skilled in the art will realize that various changes and modifications may be made to the invention without departing from the spirit of the invention, and it is intended to claim all such changes and modifications as fall within the scope of the invention.

## Claims

1. A shaving cream or gel containing microparticles.

2. A shaving cream or gel which contains additives which act to exfoliate dead skin cells and to remove accumulated debris from the surface of the skin.

3. A shaving cream or gel according to claim 1 or claim 2, wherein the microparticles are selected from the group consisting of powders, beads, microencapsulated particles or any combination thereof.

4. A shaving cream or gel according to claim 3, wherein the powders are selected from the group consisting of crushed nut shells, crushed apricot seeds, loofah, talc, magnesium stearate, chalk, kaolin, zinc oxide, titanium oxide, coated powders, beads, micro-silica beads (preferably those with 90/10 ratio), boron nitride, zinc stearate, magnesium oxide, cellulose powders, nylon powder or polyamid resin, silk powder protein, silk coated pigments, calcium carbonate, magnesium carbonate, mica, sericite, organic plastic powders, polymethylmethacrylate beads, polyethylene beads and micropowders, polyvinylidene, polytetrafluoroethylene, polyamides, polyurethane, polystyrene, synthetic clay, magnesium lithium sodium silicate and silica, silicon oxide spheres or combinations thereof.

5. A shaving cream or gel according to claim 3 or claim 4, wherein the microencapsulated particles are selected from the group consisting of microspheres, microcapsules or combinations thereof.

6. A shaving cream or gel according to any of claims 3 to 5, wherein the beads contain jojoba oil, mineral oil, sesame oil, silicon oil, aloe vera oil, tocopheryl acetate, fragrance, vitamin E, dimethicone, allantoin, lanolin, sodium PCA, color, vitamin A, vitamin C, glycolic acid, glycerin, petrolatum, propane, isobutane or combinations thereof.

7. A shaving cream or gel according to claim 5 or 6 when dependent from claim 5, wherein the microencapsulated particles contain jojoba oil, mineral oil, sesame oil, silicone oil, aloe vera oil, tocopheryl acetate, fragrance, vitamin E, dimethicone, allantoin, lanolin, sodium PCA, color, vitamin A, vitamin C, glycolic acid, glycerin, petrolatum, propane, isobutane or combinations thereof.

8. A shaving cream or gel according to any of claims 3 to 7, wherein the shaving cream or gel is dispensable as an aerosol.

9. A shaving cream or gel according to any of claims 3 to 8, wherein the shaving cream or gel is dispensable as a post-foaming gel.

10. A shaving cream or gel according to any of claims 3 to 9, wherein the shaving cream or gel is dispensable as a non-aerosol.
